# EUROPEAN PATENT APPLICATION

(11) **EP 4 740 945 A2**
(43) Date of publication of application: **13.05.2026**
(21) Application number: 25220295.7
(22) Date of filing: 24.11.2021
(51) Int. Cl.: A61K 31/465

(54) **NICOTINE-CONTAINING AGGLOMERATES AND METHODS OF FORMING THE SAME**

(30) Priority: 06.04.2021 US 202117223787
(62) Divisional of application: 21827510.5
(71) Applicant: Altria Client Services LLC, Richmond, VA 23230 (US)
(72) Inventor: Zhuang, Shuzhong, Richmond, 23219 (US); Tran, Valerie L., Richmond, 23219 (US); Yu, Shaoyong, Richmond, 23219 (US)
(74) Representative: Richardt Patentanwälte PartG mbB

(57) **Abstract**

A method for preparing nicotine-containing agglomerates includes introducing a solid particulate into a fluid bed granulator, and introducing a binder solution into the fluid bed granulator such that the binder solution contacts the solid particulate. The solid particulate and/or binder solution may be introduced into the fluid bed granulator in parts, concurrently or in series. The binder solution includes a binder material and a solvent, which may include water. Introducing the binder solution into the fluid bed granulator may include spraying the binder solution into a fluid bed of the fluid bed granulator during circulation of the fluid bed and materials therein, such as the solid particulate. The fluid bed may further include a filler material and/or an additive. The filler material and/or the additive may be introduced into the fluid bed granulator prior to, concurrently with, or subsequent to the addition of the solid particulate and/or binder solution.

## Description

### BACKGROUND

### Field

The present disclosure relates to nicotine-containing agglomerates suitable for use with or in oral products and methods of forming the same.

### Description of Related Art

Oral nicotine products are available in a variety of formats, such as chewing gums, sprays, lozenges, dissolvable tablets, non-dissolvable chews, films, gels, capsules, sticks (e.g., coated wooden dowels or singular dissolvable sticks), and pouches (e.g., containing fibers or granules). Oral products may have nicotine levels that create a familiar experience for adult tobacco consumers.

### SUMMARY

At least some example embodiments relate to a method for preparing nicotine-containing agglomerates.

In at least one example embodiment, the method for preparing nicotine-containing agglomerates includes introducing a solid particulate into a fluid bed granulator, where the solid particulate includes a nicotine-containing powder; and introducing a binder solution into the fluid bed granulator such that the binder solution contacts the solid particulate.

In at least one example embodiment, the binder solution and the solid particulate may be introduced into the fluid bed granulator simultaneously.

In at least one example embodiment, the binder solution includes a binder material. The binder material may include pyrrolidone polymers, copolymers of polyvinylpyrrolidone (PVP) and vinyl acetate, copolymers of methacrylates and acrylic acid, hydroxypropyl methylcellulose (HPMC), or any combination thereof.

In at least one example embodiment, the binder solution includes a binder material and a solvent. The solvent may include water.

In at least one example embodiment, the binder solution may be introduced downwardly from a top spray position, introduced upwardly from a bottom spray position, introduced laterally from a sideways spray, or any combination thereof.

In at least one example embodiment, the method may further include circulating the solid particulate in the fluid bed granulator, where the binder solution is introduced into the fluid bed granulator during the circulating.

In at least one example embodiment, the introducing of the solid particulate into the fluid bed granulator may include adding a first portion of the solid particulate to the fluid bed granulator and adding a second portion of the solid particulate to the fluid bed granulator during the circulating of the first portion of the solid particulate and during the introduction of the binder solution.

In at least one example embodiment, the second portion of the solid particulate may be sprayed onto other materials in the fluid bed granulator.

In at least one example embodiment, the binder solution may have a binder inlet temperature ranging from 1 °C to 99 °C, and the solid particulate may have a solid particulate inlet temperature ranging from 1 °C to 100 °C.

In at least one example embodiment, the nicotine-containing powder may include a plurality of fine particles having an average diameter ranging from 0.1 µm to 3.0 mm.

In at least one example embodiment, the method may further include pretreating the solid particulate prior to the introducing the solid particulate.

In at least one example embodiment, the pretreating may include spraying a binder material onto the nicotine-containing powder so as to pre-wet the solid particulate.

In at least one example embodiment, the pretreating may include pre-mixing the solid particulate.

In at least one example embodiment, the solid particulate may further include a filler material.

In at least one example embodiment, the method may further include contacting the filler material and the nicotine-containing powder.

In at least one example embodiment, the filler material may include a polysaccharide, a bulk sweetener, a sugar alcohol, or any combination thereof.

In at least one example embodiment, the method may further include adding a filler material into the fluid bed granulator such that the filler material contacts the solid particulate.

In at least one example embodiment, the introducing the solid particulate into the fluid bed granulator may include spraying the solid particulate onto the filler material.

In at least one example embodiment, the solid particulate may further include a flavorant, a pH modifier, an antioxidant, or any combination thereof.

In at least one example embodiment, the method may further include adding an additive into the fluid bed granulator such that the additive contacts the solid particulate, the additive including a flavorant, a pH modifier, an antioxidant, or any combination thereof.

### BRIEF DESCRIPTION OF THE DRAWING

The various features and advantages of the non-limiting embodiments herein may become more apparent upon review of the detailed description in conjunction with the accompanying drawing. The accompanying drawing is merely provided for illustrative purposes and should not be interpreted to limit the scope of the claims. The accompanying drawing is not to be considered as drawn to scale unless explicitly noted. For purposes of clarity, various dimensions of the drawing may have been exaggerated.

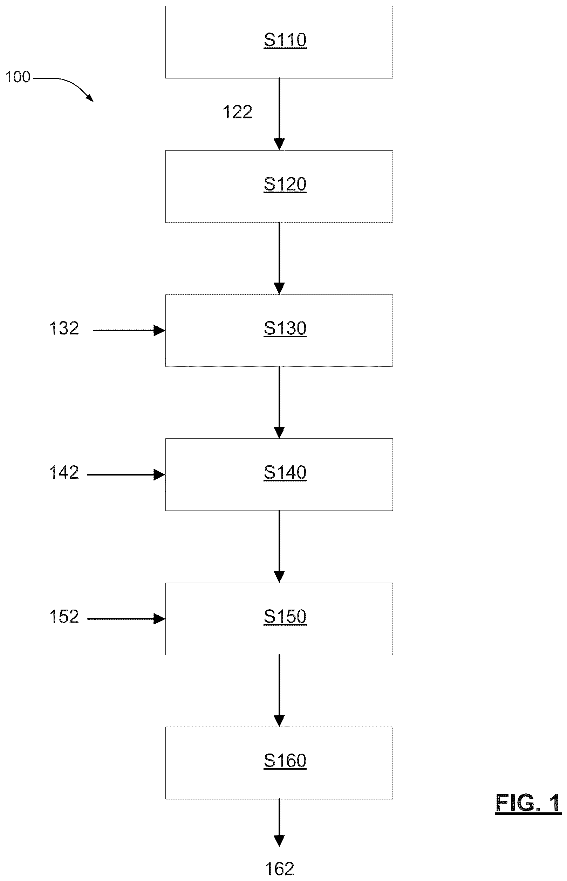

FIG. 1 is a flow diagram illustrating a method for forming a nicotine-containing agglomerate in accordance with at least one example embodiment.

### DETAILED DESCRIPTION OF EXAMPLE EMBODIMENTS

Some detailed example embodiments are disclosed herein. However, specific structural and functional details disclosed herein are merely representative for purposes of describing example embodiments. Example embodiments may, however, be embodied in many alternate forms and should not be construed as limited to only the example embodiments set forth herein.

Accordingly, while example embodiments are capable of various modifications and alternative forms, example embodiments thereof are shown by way of example in the drawings and will herein be described in detail. It should be understood, however, that there is no intent to limit example embodiments to the particular forms disclosed, but to the contrary, example embodiments are to cover all modifications, equivalents, and alternatives falling within the scope of example embodiments. Like numbers refer to like elements throughout the description of the figures.

It should be understood that when an element or layer is referred to as being "on," "connected to," "coupled to," or "covering" another element or layer, it may be directly on, connected to, coupled to, or covering the other element or layer or intervening elements or layers may be present. In contrast, when an element is referred to as being "directly on," "directly connected to," or "directly coupled to" another element or layer, there are no intervening elements or layers present. Like numbers refer to like elements throughout the specification. As used herein, the term "and/or" includes any and all combinations of one or more of the associated listed items.

It should be understood that, although the terms first, second, third, etc., may be used herein to describe various elements, regions, layers and/or sections, these elements, regions, layers, and/or sections should not be limited by these terms. These terms are only used to distinguish one element, region, layer, or section from another region, layer, or section. Thus, a first element, component, region, layer, or section discussed below could be termed a second element, region, layer, or section without departing from the teachings of example embodiments.

Spatially relative terms (e.g., "beneath," "below," "lower," "above," "upper," "inside," "outside," and the like) may be used herein for ease of description to describe one element or feature's relationship to another element(s) or feature(s) as illustrated in the figures. It should be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the term "below" may encompass both an orientation of above and below. The device may be otherwise oriented (rotated 90 degrees or at other orientations) and the spatially relative descriptors used herein interpreted accordingly.

The terminology used herein is for the purpose of describing various example embodiments only and is not intended to be limiting of example embodiments. As used herein, the singular forms "a," "an," and "the" are intended to include the plural forms as well, unless the context clearly indicates otherwise. It will be further understood that the terms "includes," "including," "comprises," and/or "comprising," specify the presence of stated features, integers, steps, operations, and/or elements, but do not preclude the presence or addition of one or more other features, integers, steps, operations, elements, and/or groups thereof.

Example embodiments are described herein with reference to cross-sectional illustrations that are schematic illustrations of example embodiments. As such, variations from the shapes of the illustrations are to be expected. Thus, example embodiments should not be construed as limited to the shapes of regions illustrated herein but are to include deviations and variations in shapes. When the terms "about" or "substantially" are used in connection with a numerical value, it is intended that the associated numerical value include a tolerance of ±10% around the stated numerical value unless the context indicates otherwise.

Unless otherwise defined, all terms (including technical and scientific terms) used herein have the same meaning as commonly understood by one of ordinary skill in the art to which example embodiments belong. It will be further understood that terms, including those defined in commonly used dictionaries, should be interpreted as having a meaning that is consistent with their meaning in the context of the relevant art and will not be interpreted in an idealized or overly formal sense unless expressly so defined herein.

At least one example embodiment relates to methods for forming nicotine-containing agglomerates suitable for inclusion in oral products. In some example embodiments, the nicotine-containing agglomerates can be prepared using fluid bed granulator techniques. Nicotine-containing agglomerates prepared using such techniques can be substantially uniform, such that the nicotine-containing agglomerates have an average particle size (90% distribution) ranging from about 50 µm to about 3,000 µm (e.g., about 100 µm to about 2,000 µm or about 500 µm to about 1,000 µm). For example, the nicotine-containing agglomerates may have an average particle size (90% distribution) greater than or equal to about 50 µm (e.g., greater than or equal to about 100 µm, greater than or equal to about 200 µm, greater than or equal to about 300 µm, greater than or equal to about 400 µm, greater than or equal to about 500 µm, greater than or equal to about 600 µm, greater than or equal to about 700 µm, greater than or equal to about 800 µm, greater than or equal to about 900 µm, greater than or equal to about 1,000 µm, greater than or equal to about 1,100 µm, greater than or equal to about 1,200 µm, greater than or equal to about 1,300 µm, greater than or equal to about 1,400 µm, greater than or equal to about 1,500 µm, greater than or equal to about 1,600 µm, greater than or equal to about 1,700 µm, greater than or equal to about 1,800 µm, greater than or equal to about 1,900 µm, greater than or equal to about 2,000 µm, greater than or equal to about 2,100 µm, greater than or equal to about 2,200 µm, greater than or equal to about 2,300 µm, greater than or equal to about 2,400 µm, greater than or equal to about 2,500 µm, greater than or equal to about 2,600 µm, greater than or equal to about 2,700 µm, greater than or equal to about 2,800 µm, or greater than or equal to about 2,900 µm). The nicotine-containing agglomerates may have an average particle size (90% distribution) less than or equal to about 3,000 µm (e.g., less than or equal to about 2,900 µm, less than or equal to about 2,800 µm, less than or equal to about 2,700 µm, less than or equal to about 2,600 µm, less than or equal to about 2,500 µm, less than or equal to about 2,400 µm, less than or equal to about 2,300 µm, less than or equal to about 2,200 µm, less than or equal to about 2,100 µm, less than or equal to about 2,000 µm, less than or equal to about 1,900 µm, less than or equal to about 1,800 µm, less than or equal to about 1,700 µm, less than or equal to about 1,600 µm, less than or equal to about 1,500 µm, less than or equal to about 1,400 µm, less than or equal to about 1,300 µm, less than or equal to about 1,200 µm, less than or equal to about 1,100 µm, less than or equal to about 1,000 µm, less than or equal to about 900 µm, less than or equal to about 800 µm, less than or equal to about 700 µm, less than or equal to about 600 µm, less than or equal to about 500 µm, less than or equal to about 400 µm, less than or equal to about 300 µm, less than or equal to about 200 µm, or less than or equal to about 100 µm).

In at least one example embodiment, nicotine-containing agglomerates may have a moisture content less than or equal to about 15 % (e.g., less than or equal to about 14 %, less than or equal to about 13 %, less than or equal to about 12 %, less than or equal to about 11 %, less than or equal to about 10 %, less than or equal to about 9 %, less than or equal to about 8 %, less than or equal to about 7 %, less than or equal to about 6 %, less than or equal to about 5 %, less than or equal to about 4 %, less than or equal to about 3 %, less than or equal to about 2 %, or less than or equal to about 1 %).

In at least one example embodiments, nicotine-containing agglomerates may have a nicotine content less than or equal to about 25 wt.% (e.g., less than or equal to about 24 wt.%, less than or equal to about 23 wt.%, less than or equal to about 22 wt.%, less than or equal to about 21 wt.%, less than or equal to about 20 wt.%, less than or equal to about 19 wt.%, less than or equal to about 18 wt.%, less than or equal to about 17 wt.%, less than or equal to about 16 wt.%, less than or equal to about 15 wt.%, less than or equal to about 14 wt.%, less than or equal to about 13 wt.%, less than or equal to about 12 wt.%, less than or equal to about 11 wt.%, less than or equal to about 10 wt.%, less than or equal to about 9 wt.%, less than or equal to about 8 wt.%, less than or equal to about 7 wt.%, less than or equal to about 6 wt.%, less than or equal to about 5 wt.%, less than or equal to about 4 wt.%, less than or equal to about 3 wt.%, less than or equal to about 2 wt.%, or less than or equal to about 1 wt.%).

In at least one example embodiment, the nicotine-containing agglomerates can include a collection or mass of a plurality of fine particles. In at least one example embodiment, the nicotine-containing agglomerates can includes a plurality of nicotine particles, as liquid or solid droplets or particles. In at least one example embodiment, each nicotine-containing agglomerate can include an amount of the nicotine particles ranging from about 0.1 wt.% to about 40 wt.%. For example, the nicotine-containing agglomerates can include greater than or equal to about 0.1 wt.% of the nicotine particles (e.g., greater than or equal to about 0.5 wt.%, greater than or equal to about 1 wt.%, greater than or equal to about 5 wt.%, greater than or equal to about 10 wt.%, greater than or equal to about 15 wt.%, greater than or equal to about 20 wt.%, greater than or equal to about 25 wt.%, greater than or equal to about 30 wt.%, or greater than or equal to about 35 wt.%). The nicotine-containing agglomerates can include less than or equal to about 40 wt.% (e.g., less than or equal to about 35 wt.%, less than or equal to about 30 wt.%, less than or equal to about 25 wt.%, less than or equal to about 20 wt.%, less than or equal to about 15 wt.%, less than or equal to about 10 wt.%, less than or equal to about 5 wt.%, less than or equal to about 1 wt.%, or less than or equal to about 0.5 wt.%).

In at least one example embodiment, the nicotine particles may have an average particle size (90% distribution) ranging from about 0.1 µm to about 3 mm. For example, the nicotine particles may have an average particle size greater than or equal to about 0.1 µm (*e.g.,* greater than or equal to about 0.2 µm, greater than or equal to about 0.5 µm, greater than or equal to about 1 µm, greater than or equal to about 1.5 µm, greater than or equal to about 2 µm, or greater than or equal to about 2.5 µm). The nicotine particles may have an average particle size less than or equal to about 3 mm (e.g., less than or equal to about 2.9 mm, less than or equal to about 2.8 mm, less than or equal to about 2.5 mm, less than or equal to about 2 mm, less than or equal to about 1.5 µm, less than or equal to about 1 µm, less than or equal to about 0.5 µm, or less than or equal to about 0.2 µm).

In at least one example embodiment, the nicotine particles includes nicotine, a nicotine complex (such as, nicotine polacrilex), a nicotine salt, or any combination thereof. The nicotine salt may include, for example, citrate, monotartrate, bitartrate, bitartrate dihydrate, salicylate, sulfate, bisulfate, phosphate, acid phosphate, acetate, lactate, succinate, maleate, fumarate, gluconate, saccharate, benzoate, methanesulfonate, hydrochloride, hydrobromide, hydroiodide, or any combination thereof.

In at least one example embodiment, the nicotine-containing agglomerates can include a binder material. For example, each nicotine-containing agglomerate can include less than or equal to about 40 wt.% of the binder material (e.g., less than or equal to about 35 wt.%, less than or equal to about 30 wt.%, less than or equal to about 25 wt.%, less than or equal to about 20 wt.%, less than or equal to about 15 wt.%, less than or equal to about 10 wt.%, less than or equal to about 5 wt.%, less than or equal to about 4 wt.%, less than or equal to about 3 wt.%, less than or equal to about 2 wt.%, or less than or equal to about 1 wt.%). In at least one example embodiment, the binder material includes pyrrolidone polymers, copolymers of polyvinylpyrrolidone (PVP) and vinyl acetate, copolymers of methacrylates and acrylic acid, hydroxypropyl methylcellulose (HPMC), or any combination thereof.

In at least one example embodiment, the nicotine-containing agglomerates can include a filler material. For example, each nicotine-containing agglomerate can include less than or equal to about 99 wt.% of the filler material (e.g., less than or equal to about 95 wt.%, less than or equal to about 90 wt.%, less than or equal to about 85 wt.%, less than or equal to about 80 wt.%, less than or equal to about 75 wt.%, less than or equal to about 70 wt.%, less than or equal to about 65 wt.%, less than or equal to about 60 wt.%, less than or equal to about 55 wt.%, less than or equal to about 50 wt.%, less than or equal to about 45 wt.%, less than or equal to about 40 wt.%, less than or equal to about 35 wt.%, less than or equal to about 30 wt.%, less than or equal to about 25 wt.%, less than or equal to about 20 wt.%, less than or equal to about 15 wt.%, less than or equal to about 10 wt.%, less than or equal to about 5 wt.%, or less than or equal to about 1 wt.%).

In at least one example embodiment, the filler material includes a polysaccharide, a bulk sweetener, a sugar alcohol, or any combination thereof. The polysaccharide may include microcrystalline cellulose, methyl cellulose, hydroxyl propyl cellulose, hydroxyl methyl propyl cellulose, pectin, carboxyl methyl cellulose, dextrin, maltodextrin, xanthan gum, agar, carrageenan, guar gum, alginate, or any combination thereof. The bulk sweetener may include sucrose, dextrose, fructose, lactose, faffinose, trehalose, maltose, maltodextrins, or any combination thereof. The sugar alcohol may include sorbitol, mannitol, xylitol, maltitol, lactitol, isomalt, or any combination thereof.

In at least one example embodiment, the nicotine-containing agglomerates can include an additive. For example, each nicotine-containing agglomerate can include an amount of the additive ranging from about 0.1 wt.% to about 40 wt.%. For example, each nicotine-containing agglomerate may include greater than or equal to about 0.1 wt.% of the additive (e.g., greater than or equal to about 0.5 wt.%, greater than or equal to about 1 wt.%, greater than or equal to about 5 wt.%, greater than or equal to about 10 wt.%, greater than or equal to about 15 wt.%, greater than or equal to about 20 wt.%, greater than or equal to about 25 wt.%, greater than or equal to about 30 wt.%, or greater than or equal to about 35 wt.%). Each nicotine-containing agglomerate may include less than or equal to about 40 wt.% of the additive (e.g., less than or equal to about 35 wt.%, less than or equal to about 30 wt.%, less than or equal to about 25 wt.%, less than or equal to about 20 wt.%, less than or equal to about 15 wt.%, less than or equal to about 10 wt.%, less than or equal to about 5 wt.%, or less than or equal to about 1 wt.%).

In at least one example embodiment, the additive includes a flavorant, a pH modifier or adjuster, an antioxidant, or any combination thereof.

In at least one example embodiment, each nicotine-containing agglomerate can include an amount of the flavorant ranging from 0 wt.% to about 30 wt.%. For example, each nicotine-containing agglomerate can include greater than or equal to about 0 wt.% of the flavorant (e.g., greater than or equal to about 0.1 wt.%, greater than or equal to about 0.5 wt.%, greater than or equal to about 1 wt.%, greater than or equal to about 5 wt.%, greater than or equal to about 10 wt.%, greater than or equal to about 15 wt.%, greater than or equal to about 20 wt.%, or greater than or equal to about 25 wt.%). Each nicotine-containing agglomerate can include less than or equal to about 30 wt% of the flavorant (e.g., less than or equal to about 25 wt%, less than or equal to about 20 wt%, less than or equal to about 15 wt%, less than or equal to about 10 wt%, less than or equal to about 5 wt%, less than or equal to about 1 wt%, or less than or equal to about 0.5 wt%).

In at least one example embodiment, the flavorant can include peppermint, spearmint, wintergreen, menthol, cinnamon, chocolate, vanillin, licorice, clove, anise, sandalwood, geranium, rose oil, vanilla, lemon oil, cassia, fennel, ginger, ethylacetate, isoamylacetate, propylisobutyrate, isobutylbutyrate, ethylbutyrate, ethylvalerate, benzylformate, limonene, cymene, pinene, linalool, geraniol, citronellol, citral, orange oil, coriander oil, borneol, fruit extract, coffee, tea, cacao, mint, pomegranate, acai, raspberry, blueberry, strawberry, boysenberry, cranberry, bourbon, scotch, whiskey, cognac, hydrangea, lavender, apple, peach, pear, cherry, plum, orange, lime, lichy, grape, grapefruit, butter, rum, coconut, almond, pecan, walnut, hazelnut, french vanilla, macadamia, sugar cane, maple, cassis, caramel, banana, malt, espresso, kahlua, white chocolate, spice flavors such as cinnamon, clove, cilantro, basil, oregano, garlic, mustard, nutmeg, rosemary, thyme, tarragon, dill, sage, anise, and fennel, methyl salicylate, linalool, jasmine, coffee, olive oil, sesame oil, sunflower oil, bergamot oil, geranium oil, lemon oil, ginger oil, balsamic vinegar, rice wine vinegar, and red wine vinegar, all spice, pimento, mango, soursop, sweetsop, naseberry, sorrel, or any combination thereof. In at least one example embodiment, the flavorant may be an encapsulated flavorant.

In at least one example embodiment, each nicotine-containing agglomerate can include an amount of the pH modifier ranging from 0 wt.% to about 10 wt.%. For example, each nicotine-containing agglomerate can include greater than or equal to about 0 wt.% of the pH modifier (e.g., greater than or equal to about 0.1 wt.%, greater than or equal to about 0.5 wt.%, greater than or equal to about 1 wt.%, greater than or equal to about 2 wt.%, greater than or equal to about 3 wt.%, greater than or equal to about 4 wt.%, greater than or equal to about 5 wt.%, greater than or equal to about 6 wt.%, greater than or equal to about 7 wt.%, greater than or equal to about 8 wt.%, or greater than or equal to about 9 wt.%). Each nicotine-containing agglomerate can include less than or equal to about 10 wt.% of the pH modifier (e.g., less than or equal to about 9 wt.%; less than or equal to about 8 wt.%, less than or equal to about 7 wt.%, less than or equal to about 6 wt.%, less than or equal to about 5 wt.%, less than or equal to about 4 wt.%, less than or equal to about 3 wt.%, less than or equal to about 2 wt.%, less than or equal to about 1 wt.%, less than or equal to about 0.5 wt.%, or less than or equal to about 0.1 wt.%).

In at least one example embodiment, the pH modifier can include ammonium carbonate, sodium carbonate, sodium bicarbonate, potassium carbonate, potassium bicarbonate, or any combination thereof.

In at least one example embodiment, each nicotine-containing agglomerate can include an amount of the antioxidant ranging from 0 wt.% to about 10 wt.%. For example, each nicotine-containing agglomerate can include greater than or equal to about 0 wt.% of the antioxidant (e.g., greater than or equal to about 0.1 wt.%, greater than or equal to about 0.5 wt.%, greater than or equal to about 1 wt.%, greater than or equal to about 2 wt.%, greater than or equal to about 3 wt.%, greater than or equal to about 4 wt.%, greater than or equal to about 5 wt.%, greater than or equal to about 6 wt.%, greater than or equal to about 7 wt.%, greater than or equal to about 8 wt.%, or greater than or equal to about 9 wt.%). Each nicotine-containing agglomerate can include less than or equal to about 10 wt.% of the antioxidant (e.g., less than or equal to about 9 wt.%; less than or equal to about 8 wt.%, less than or equal to about 7 wt.%, less than or equal to about 6 wt.%, less than or equal to about 5 wt.%, less than or equal to about 4 wt.%, less than or equal to about 3 wt.%, less than or equal to about 2 wt.%, less than or equal to about 1 wt.%, less than or equal to about 0.5 wt.%, or less than or equal to about 0.1 wt.%).

In at least one example embodiment, the antioxidant includes ascorbyl palmitate, tertiary butylhydroquinone (TBHQ), butylated hydroxytoulene (BHT), ascorbic acid, sodium ascorbate, monosterol citrate, tocopherols, propyl gallate, or any combination thereof.

FIG. 1 is a flow diagram illustrating a method for forming a nicotine-containing agglomerate in accordance with at least one example embodiment.

In some example embodiments, a method for forming such nicotine-containing agglomerates generally includes heating and cooling a first mixture, adding a nicotine-containing material to the cooled first mixture to form a second mixture, and cooling the second mixture to a further temperature to form solidified structures, such as one or more sheets. The one or more solidified structures can be fragmented to form the encapsulated nicotine granules for inclusion in oral products and having desired sensory characteristics.

In at least one example embodiment, as shown in FIG. 1, a method 100 for preparing nicotine-containing agglomerates 162 for inclusion in oral products such as gums, sprays, lozenges, dissolvable tablets, non-dissolvable chews, films, gels, capsules, and pouches (e.g., containing fibers or granules). The nicotine-containing agglomerates 162 may each include a plurality of nicotine particles, such as detailed above. The method 100 can be performed using a batch process, a continuous process, or both a batch process and a continuous process.

In at least one example embodiment, the method 100 for preparing nicotine-containing agglomerates 162 includes introducing S120 a solid particulate 122 into a fluid bed granulator. In at least one example embodiment, introducing S120 the solid particulate 122 into the fluid bed granulator includes introducing or adding the solid particulate 122, or a portion of the solid particulate 122, into, for example, a screw-feeder of the fluid bed granulator, and thereafter, moving (e.g., feeding) the solid particulate 122 from the screw-feeder to a fluid bed of the fluid bed granulator using a bulk process or a step-wise process. In at least one example embodiment, introducing S120 the solid particulate 122 into the fluid bed granulator includes adding the solid particulate 122 directly to the fluid bed of the fluid bed granulator using a bulk process or a step-wise process, for example at the beginning or start of the process 100. In still other example embodiments, introducing S120 the solid particulate 122 into the fluid bed granulator includes spraying the solid particulate 122, or a portion of the solid particulate 122, onto other materials such as discussed below, including for example, a binder material, a filler material, and/or other additive materials (such as a flavorant, a pH modifier, an antioxidant, or any combination thereof).

In at least one example embodiment, a temperature of the solid particulate 122 may, upon introduction S120, range from about 1 °C to about 100 °C. For example, the solid particulate 122 may have a solid particulate inlet temperature greater than or equal to about 1 °C (e.g., greater than or equal to about 5°C, greater than or equal to about 10 °C, greater than or equal to about 20 °C, greater than or equal to about 30 °C, greater than or equal to about 40 °C, greater than or equal to about 50 °C, greater than or equal to about 60 °C, greater than or equal to about 70 °C, greater than or equal to about 80 °C, greater than or equal to about 90 °C, or greater than or equal to about 95 °C). The solid particulate 122 may have a solid particulate inlet temperature less than or equal to about 100 °C (e.g., less than or equal to about 95 °C, less than or equal to about 90 °C, less than or equal to about 80 °C, less than or equal to about 70 °C, less than or equal to about 60 °C, less than or equal to about 50 °C, less than or equal to about 40 °C, less than or equal to about 30 °C, less than or equal to about 20 °C, less than or equal to about 10 °C, or less than or equal to about 5 °C). Modifying the solid particulate inlet temperature may modify the characteristics of the formed nicotine-containing agglomerates 162. For example, in at least one example embodiment, modifying the solid particulate inlet temperature may impact the stickiness of the solid particulates, and therefore, how the particles are bound together during the granulation process.

The solid particulate 122 includes a nicotine-containing powder, and the nicotine-containing powder includes a plurality of fine particles (or nicotine particles), such as described above. The fine particles may have, for example, an average particle size (90% distribution) ranging from about 0.1 µm to about 3 mm, such as described above.

In some example embodiments, the solid particulate 122 further includes a filler material, as described above. For example, the solid particulate 122 may include less than or equal to about 99.99 wt.% of the filler material (e.g., less than or equal to about 90 wt.%, less than or equal to about 85 wt.%, less than or equal to about 80 wt.%, less than or equal to about 75 wt.%, less than or equal to about 70 wt.%, less than or equal to about 65 wt.%, less than or equal to about 60 wt.%, less than or equal to about 55 wt.%, less than or equal to about 50 wt.%, less than or equal to about 45 wt.%, less than or equal to about 40 wt.%, less than or equal to about 35 wt.%, less than or equal to about 30 wt.%, less than or equal to about 25 wt.%, less than or equal to about 20 wt.%, less than or equal to about 15 wt.%, less than or equal to about 10 wt.%, or less than or equal to about 5 wt.%). Although not illustrated, in some example embodiments, the method 100 may include contacting the filler and the nicotine-containing powder so to form the solid particulate 122.

In some example embodiments, the solid particulate 122 further includes an additive, as described above. For example, the solid particulate 122 may include can include an amount of the additive ranging from about 0.1 wt.% to about 40 wt.%. Although not illustrated, in some example embodiments, the method 100 may include contacting the filler and the nicotine-containing powder so to form the solid particulate 122. As described above, in at least one example embodiment, the additive includes a flavorant, a pH modifier, an antioxidant, or any combination thereof.

In at least one example embodiment, the method 100 includes pre-processing or pre-treating S110 the solid particulate 122. Pre-processing or pre-treating S110 the solid particulate 122 may include, for example, pre-mixing, pre-wetting, fluidity improvement, particle size variation, or any combination thereof.

In at least one example embodiment, the pre-processing or pre-treating S110 includes pre-wetting the solid particulate 122. Pre-wetting the solid particulate 122 may include contacting the solid particulate 122 and a (first) binder material, such that the solid particulate 122 includes less than or equal to about 40 wt.% of the binder material (e.g., (e.g., less than or equal to about 35 wt.%, less than or equal to about 30 wt.%, less than or equal to about 25 wt.%, less than or equal to about 20 wt.%, less than or equal to about 15 wt.%, less than or equal to about 10 wt.%, less than or equal to about 5 wt.%, less than or equal to about 1 wt.%, or less than or equal to about 0.5 wt.%).

In at least one example embodiment, the (first) binder material may be sprayed onto the nicotine-containing powder. The (first) binder material may include pyrrolidone polymers, copolymers of polyvinylpyrrolidone (PVP) and vinyl acetate, copolymers of methacrylates and acrylic acid, hydroxypropyl methylcellulose (HPMC), or any combination thereof. Pre-wetting the solid particulate 122 may improve subsequent granulation, for example by promoting agglomeration formation and/or reducing (or minimizing) ingredient loss.

In at least one example embodiment, the pre-processing or pre-treating S110 includes pre-mixing the solid particulate 122. Pre-mixing the solid particulate 122 may include mixing the solid particulate 122 using, for example, a screw blender, a planetary blender, a v-blender, a ribbon blender, a high shear mixer, or a combination thereof. Pre-mixing the solid particulate 122 may enhance the uniformity of the solid particulate 122.

In at least one example embodiment, the pre-processing or pre-treating S110 includes improving the fluidity of the solid particulate 122. The fluidity of the solid particulate 122 may be improved by subjecting the solid particulate 122 to a roller-compaction process (for example, to increase the density of the solid particulate 122), a spray-drying/chilling process (for example, to form a dry powder), a extrusion-marumerization/spheronization process (for example, to create solid particulate 122 having a fixed cross section and/or to form solid particulate 122 having a small rounded or spherical granule shape), other like industrial process, or any combination thereof. Increasing the fluidity of the solid particulate 122 may help to facilitate the granulation process.

In at least one example embodiment, the method 100 includes introducing S130 a binder solution 132 into the fluid bed granulator. For example, the binder solution 132 may be introduced S130 in such a manner that at least a portion of the binder solution 132 contacts at least a portion of the solid particulate 122, so as to fluidize the solid particulate 122.

Like the introduction S120 of the solid particulate 122, in at least one example embodiment, the binder solution 132, or a portion of the binder solution 132, may be introduced S130 directly to the fluid bed of the fluid bed granulator using a bulk process or a step-wise process, for example only, at the beginning or start of the process 100 and/or concurrently with or subsequently to the introduction S120 of the solid particulate 122. For example, the binder solution 132 may be sprayed into the fluid bed of the fluid bed granulator. The binder solution 132 may be sprayed into the fluid bed. For example, in some example embodiments, the binder solution 132 may be sprayed onto the solid particulate 122 that is in the fluid bed. In at least one example embodiment, the binder solution 132 may be sprayed in a downwardly fashion, for example, from a top spray position; in an upwardly fashion, for example, from a bottom spray position; a sideway fashion, for example, from a side spray position; or any combination thereof. Modifying the spray position, as well as the spray pattern and rate, may modify the characteristics of the formed nicotine-containing agglomerates 162, for example only, the particle size and density of the nicotine-containing agglomerates 162. For example, in at least one example embodiment, increasing the air flow may favor smaller particle sizes. In at least one example embodiment, increasing temperature may reduce processing time.

In at least one example embodiment, a temperature of the binder solution 132 upon introduction S130 of the binder solution 132 into the fluid bed granulator ranges from about 1 °C to about 99 °C. For example, the binder solution may have a binder inlet temperature greater than or equal to about 1 °C (e.g., greater than or equal to about 5 °C, greater than or equal to about 10 °C, greater than or equal to about 20 °C, greater than or equal to about 30 °C, greater than or equal to about 40 °C, greater than or equal to about 50 °C, greater than or equal to about 60 °C, greater than or equal to about 70 °C, greater than or equal to about 80 °C, greater than or equal to about 85 °C, greater than or equal to about 90 °C, or greater than or equal to about 95 °C).The binder solution may have a binder inlet temperature less than or equal to about 99 °C (e.g., less than or equal to about 95 °C, less than or equal to about 90 °C, less than or equal to about 85 °C, less than or equal to about 80 °C, less than or equal to about 70 °C, less than or equal to about 60 °C, less than or equal to about 50 °C, less than or equal to about 40 °C, less than or equal to about 30 °C, less than or equal to about 20 °C, less than or equal to about 10 °C, or less than or equal to about 5 °C). Modifying the binder inlet temperature may modify the characteristics of the binder solution 132, such as viscosity and fluidity, and may also impact the spray pattern and the characteristics of the formed nicotine-containing agglomerates 162.

In at least one example embodiment, the binder solution 132 includes a (second) binder material and a solvent. In at least one example embodiment, the (second) binder solution 132 may include an amount of the (second) binder material ranging from about 0.05 wt.% to about 50 wt.%, and an amount of the solvent ranging from about 60 wt.% to about 99.95 wt.%. For example, the (second) binder solution 132 may include greater than or equal to about 0.05 wt.% of the (second) binder material (e.g., greater than or equal to about 0.1 wt.%, greater than or equal to about 0.5 wt.%, greater than or equal to about 1 wt.%, greater than or equal to about 5 wt.%, greater than or equal to about 10 wt.%, greater than or equal to about 15 wt.%, greater than or equal to about 20 wt.%, greater than or equal to about 25 wt.%, greater than or equal to about 30 wt.%, greater than or equal to about 35 wt.%, greater than or equal to about 40 wt.%, or greater than or equal to about 50 wt.%. The (second) binder solution 132 may include less than or equal to about 50 wt.% of the (second) binder material (e.g., less than or equal to about 45 wt.%, less than or equal to about 40 wt.%, less than or equal to about 35 wt.%, less than or equal to about 30 wt.%, less than or equal to about 25 wt.%, less than or equal to about 20 wt.%, less than or equal to about 15 wt.%, less than or equal to about 10 wt.%, less than or equal to about 5 wt.%, less than or equal to 1 wt.%, less than or equal to about 0.5 wt.%, or less than or equal to about 0.1 wt.%). The (second) binder solution 132 may include greater than or equal to about 60 wt.% of the solvent (e.g., greater than or equal to about 65 wt.%, greater than or equal to about 70 wt.%, greater than or equal to about 75 wt.%, greater than or equal to about 80 wt.%, greater than or equal to about 85 wt.%, greater than or equal to about 90 wt.%, greater than or equal to about 95 wt.%, or greater than or equal to about 97 wt.%). The (second) binder solution 132 may include less than or equal to about 99.95 wt.% of the solvent (e.g., less than or equal to about 99 wt.%, less than or equal to about 97 wt.%, less than or equal to about 95 wt.%, less than or equal to about 90 wt.%, less than or equal to about 85 wt.%, less than or equal to about 80 wt.%, less than or equal to about 75 wt.%, less than or equal to about 70 wt.%, or less than or equal to about 65 wt.%).

In at least one example embodiment, the binder solution 132, including the (second) binder material and the solvent, may be a homogeneous liquid mixture. Although not illustrated, in some example embodiments, the method 100 may include homogenizing the binder solution 132.

In at least one example embodiment, the (second) binder material may include pyrrolidone polymers, copolymers of polyvinylpyrrolidone (PVP) and vinyl acetate, copolymers of methacrylates and acrylic acid, hydroxypropyl methylcellulose (HPMC), or any combination thereof. In at least one example embodiment, the solvent may include water. In other embodiments, the solvent may include water, ethanol, methanol, propanol, hexane, or any combination thereof.

In some example embodiments, the method 100 includes introducing S140 a filler material 142 into the fluid bed granulator. For example, the filler material 142 may be introduced S140 in such a manner that at least a portion of the filler material 142 contacts at least a portion of the solid particulate 112 and/or binder solution 132. In at least one example embodiment, the solid particulate 122 includes a first filler material and a second filler material 142 may be added into the fluid bed granulator.

Like the introduction S120 of the solid particulate 122 and/or the introduction S130 of the binder solution 132, in some example embodiments, the filler material 142, or a portion of the filler material 142, may be introduced S140 directly to the fluid bed of the fluid bed granulator using a bulk process or a step-wise process, for example only, concurrently with or subsequently to the introduction S120 of the solid particulate 122 and/or introduction S130 of the binder solution 132. In at least one example embodiment, introducing S140 the filler material 142 into the fluid bed granulator includes introducing or adding the filler material 142, or a portion of the filler material 142, into, for example, a screw-feeder of the fluid bed granulator, and thereafter, moving (e.g., feeding) the filler material from the screw-feeder to a fluid bed of the fluid bed granulator using a bulk process or a step-wise process. The filler material 142 may include a polysaccharide, a bulk sweetener, a sugar alcohol, or any combination thereof, such as described above.

In at least one example embodiment, a temperature of the filler material 142 upon the introduction S140 of the filler material 142 into the fluid bed granulator ranges from about 1 °C to about 99 °C. For example, the filler material 142 may have an inlet temperature greater than or equal to about 1 °C (e.g., greater than or equal to about 5 °C, greater than or equal to about 10 °C, greater than or equal to about 20 °C, greater than or equal to about 30 °C, greater than or equal to about 40 °C, greater than or equal to about 50 °C, greater than or equal to about 60 °C, greater than or equal to about 70 °C, greater than or equal to about 80 °C, greater than or equal to about 85 °C, greater than or equal to about 90 °C, or greater than or equal to about 95 °C). The filler material 142 may have an inlet temperature less than or equal to about 99 °C (e.g., less than or equal to about 95 °C, less than or equal to about 90 °C, less than or equal to about 85 °C, less than or equal to about 80 °C, less than or equal to about 70 °C, less than or equal to about 60 °C, less than or equal to about 50 °C, less than or equal to about 40 °C, less than or equal to about 30 °C, less than or equal to about 20 °C, less than or equal to about 10 °C, or less than or equal to about 5 °C).

In at least one example embodiment, the method 100 includes introducing S150 an additive 152 into the fluid bed granulator. For example, the additive 152 may be introduced S150 in such a manner that at least a portion of the additive 152 contacts at least a portion of the solid particulate 112 and/or binder solution 132. In at least one example embodiment, the solid particulate 122 includes a first additive and a second additive 152 may be added into the fluid bed granulator. Like the introduction S120 of the solid particulate 122 and/or the introduction S130 of the binder solution 132, in some example embodiments, the additive 152, of a portion of the additive 152, may be introduced S150 directly to the fluid bed of the fluid bed granulator using a bulk process or a step-wise process, for example only, concurrently with or subsequently to the introduction S120 of the solid particulate 122 and/or the introduction S130 of the binder solution 132 and/or the introduction S140 of the filler material 142.

In at least one example embodiment, a temperature of the additive 152 upon the introduction S150 of the additive 152 into the fluid bed granulator ranges from about 1 °C to about 99 °C. For example, the additive 152 may have an inlet temperature greater than or equal to about 1 °C (e.g., greater than or equal to about 5 °C, greater than or equal to about 10 °C, greater than or equal to about 20 °C, greater than or equal to about 30 °C, greater than or equal to about 40 °C, greater than or equal to about 50 °C, greater than or equal to about 60 °C, greater than or equal to about 70 °C, greater than or equal to about 80 °C, greater than or equal to about 85 °C, greater than or equal to about 90 °C, or greater than or equal to about 95 °C). The additive 152 may have an inlet temperature less than or equal to about 99 °C (e.g., less than or equal to about 95 °C, less than or equal to about 90 °C, less than or equal to about 85 °C, less than or equal to about 80 °C, less than or equal to about 70 °C, less than or equal to about 60 °C, less than or equal to about 50 °C, less than or equal to about 40 °C, less than or equal to about 30 °C, less than or equal to about 20 °C, less than or equal to about 10 °C, or less than or equal to about 5 °C).

In at least one example embodiments, the additive 152 includes a flavorant, a pH modifier, an antioxidant, or any combination thereof, such as described above. Introducing the additive 152 may include concurrently or subsequently adding the flavorant, the pH modifier, the antioxidant, or the combination thereof. Although not illustrated, in some example embodiments, the method 100 may include mixing the flavorant and/or the pH modifier and/or the antioxidant so to form the additive 152.

Although not specifically illustrated, the skilled artisan will appreciate that the introduction S120 of the solid particulate 122, the introduction S130 of the binder solution 132, the introduction S140 of the filler material 142, and/or the introduction S150 of the additive 152 into the fluid bed granulator may occur concurrently or in series, and that a portion or portions of the solid particulate 122, a portion or portions of the binder solution 132, a portion or portions of the filler material 142, and/or a portion or portions of the additive 152 may be added concurrently or in series. For example, in at least some example embodiments, a first portion of the solid particulate 122 may be added to the fluid bed granulator, and subsequently, a second portion of the solid particulate 122 may be added to the fluid bed granulator (for example, using a spraying process). Introduction of the solid particulate 122, the binder solution 132, the filler material 142, and/or the additive 152, and relationship therebetween, may be selected so as to prepare the nicotine-containing agglomerates 162 having desired physical and sensory characteristics.

In at least one example embodiment, the method 100 includes circulating S160 the fluid bed, including the solid particulate 122, the binder solution 132, the filler material 142, and/or the additive 152, so as to form the nicotine-containing agglomerates 162. In at least one example embodiment, the flow rate during circulating S160 may be adjusted so as to vary the density of the nicotine-containing agglomerates 162. In at least one example embodiment, the duration of the circulating S160 may be adjusted so as to vary the moisture content of the nicotine-containing agglomerates 162.

The nicotine-containing agglomerates, like those discussed above, are suitable for inclusion in oral products such as gums, sprays, lozenges, dissolvable tablets, non-dissolvable chews, films, gels, capsules, and pouches (e.g., containing fibers or granules).

In at least one example embodiment, the oral product is an oral tobacco product, an oral non-tobacco product, an oral cannabis product, or any combination thereof. The oral product may be in a form of loose material (e.g., loose cellulosic material), shaped material (e.g., plugs or twists), pouched material, tablets, lozenges, chews, gums, films, any other oral product, or any combination thereof.

In at least one example embodiment, the oral product may include chewing tobacco, snus, moist snuff tobacco, dry snuff tobacco, other smokeless tobacco and non-tobacco products for oral consumption, or any combination thereof.

While some example embodiments have been disclosed herein, it should be understood that other variations may be possible. Such variations are not to be regarded as a departure from the spirit and scope of the present disclosure, and all such modifications as would be obvious to one skilled in the art are intended to be included within the scope of the following claims.

Although described with reference to specific examples and drawings, modifications, additions and substitutions of example embodiments may be variously made according to the description by those of ordinary skill in the art. For example, the described techniques may be performed in an order different with that of the methods described, and/or elements such as the described system, architecture, devices, circuit, and the like, may be connected or combined to be different from the above-described methods, or results may be appropriately achieved by other elements or equivalents. The invention may further be described without limitation and by way of example only by the following embodiments.
1. A method for preparing nicotine-containing agglomerates, the method comprising:
   introducing a solid particulate into a fluid bed granulator, the solid particulate including a nicotine-containing powder; and
   introducing a binder solution into the fluid bed granulator such that the binder solution contacts the solid particulate.
2. The method of claim 1, wherein the binder solution and the solid particulate are introduced into the fluid bed granulator simultaneously.
3. The method of claim 1, wherein the binder solution includes a binder material, the binder material including pyrrolidone polymers, copolymers of polyvinylpyrrolidone (PVP) and vinyl acetate, copolymers of methacrylates and acrylic acid, hydroxypropyl methylcellulose (HPMC), or any combination thereof.
4. The method of claim 1, wherein the binder solution includes a binder material and a solvent, the solvent including water.
5. The method of claim 1, wherein the binder solution is introduced downwardly from a top spray position, introduced upwardly from a bottom spray position, introduced laterally from a sideways spray, or any combination thereof.
6. The method of claim 1, further including:
   circulating the solid particulate in the fluid bed granulator, wherein the binder solution is introduced into the fluid bed granulator during the circulating.
7. The method of claim 6, wherein the introducing of the solid particulate into the fluid bed granulator includes adding a first portion of the solid particulate to the fluid bed granulator and adding a second portion of the solid particulate to the fluid bed granulator during the circulating of the first portion of the solid particulate and during the introduction of the binder solution.
8. The method of claim 7, wherein the second portion of the solid particulate is sprayed onto other materials in the fluid bed granulator.
9. The method of claim 1, wherein the binder solution has a binder inlet temperature ranging from 1 °C to 99 °C, and the solid particulate has a solid particulate inlet temperature ranging from 1 °C to 100 °C.
10. The method of claim 1, wherein the nicotine-containing powder includes a plurality of fine particles having an average diameter ranging from 0.1 µm to 3 mm.
11. The method of claim 1, further including:
   pretreating the solid particulate prior to the introducing the solid particulate.
12. The method of claim 11, wherein the pretreating includes spraying a binder material onto the nicotine-containing powder so as to pre-wet the solid particulate.
13. The method of claim 11, wherein the pretreating includes pre-mixing the solid particulate.
14. The method of claim 1, wherein the solid particulate further includes a filler material.
15. The method of claim 14, further including:
   contacting the filler material and the nicotine-containing powder.
16. The method of claim 14, wherein the filler material comprises a polysaccharide, a bulk sweetener, a sugar alcohol, or any combination thereof.
17. The method of claim 1, further including:
   adding a filler material into the fluid bed granulator such that the filler material contacts the solid particulate.
18. The method of claim 17, wherein the introducing the solid particulate into the fluid bed granulator includes spraying the solid particulate onto the filler material.
19. The method of claim 1, wherein the solid particulate further comprises a flavorant, a pH modifier, an antioxidant, or any combination thereof.
20. The method of claim 1, further comprising:
   adding an additive into the fluid bed granulator such that the additive contacts the solid particulate, the additive including a flavorant, a pH modifier, an antioxidant, or any combination thereof.

## Claims

1. A method for preparing nicotine-containing agglomerates, the method comprising:
circulating a first solid particulate in a fluid bed granulator, the first solid particulate including a first nicotine-containing powder; and
introducing a second solid particulate and a binder concurrently into the fluid bed granulator during the circulating, the second solid particulate including a second nicotine-containing powder.

2. The method of claim 1, wherein the second solid particulate is sprayed onto the first solid particulate.

3. The method of any one of claims 1 or 2, wherein at least one of the first nicotine-containing powder and the second nicotine-containing powder includes a plurality of fine particles having an average diameter ranging from 0.1 micrometers to 3 millimeters.

4. The method of any one of the preceding claims, further comprising:
introducing the first solid particulate into the fluid bed granulator, wherein a solid particulate inlet temperature as the first solid particulate is introduced into the fluid bed granulator ranges from greater than or equal to about 1 °C to less than or equal to about 100 °C, and/or wherein a solid particulate inlet temperature as the second solid particulate is introduced into the fluid bed granulator ranges from greater than or equal to about 1 °C to less than or equal to about 100 °C, and/or wherein a binder inlet temperature as the binder is introduced into the fluid bed granulator ranges from greater than or equal to about 1 °C to less than or equal to about 99 °C.

5. The method of any one of the preceding claims, wherein the binder is introduced downwardly from a top spray position, introduced upwardly from a bottom spray position, introduced laterally from a sideways spray, or any combination thereof.

6. The method of any one of the preceding claims, wherein the binder includes pyrrolidone polymers, copolymers of polyvinylpyrrolidone (PVP) and vinyl acetate, copolymers of methacrylates and acrylic acid, hydroxypropyl methylcellulose (HPMC), or any combination thereof; and/or wherein the binder is an aqueous solution.

7. The method of any one of the preceding claims, wherein the binder is a first binder and the method further comprises:
pre-wetting the first and/or second solid particulate by spraying a second binder thereon, in particular spraying the binder material onto the respective nicotine-containing powder.

8. The method of any one of the preceding claims, wherein the first solid particulate further includes a filler material, optionally including a polysaccharide, a bulk sweetener, a sugar alcohol, or any combination thereof.

9. The method of claim 8, further comprising:
contacting the filler material and the first solid particulate.

10. The method of claim 9, wherein the contacting of the filler material and the first solid particulate includes adding the filler material into the fluid bed granulator such that the filler material contacts the first solid particulate.

11. The method of any one of claims 9 or 10, wherein the contacting of the filler material and the first solid particulate includes spraying the first solid particulate onto the filler material as disposed in the fluid bed granulator.

12. The method of any one of the preceding claims, wherein at least one of the first solid particulate and the second solid particulate further comprises a flavorant, a pH modifier, an antioxidant, or any combination thereof.

13. The method of any one of the preceding claims, further comprising:
adding an additive into the fluid bed granulator such that the additive contacts at least one of the first solid particulate and the second solid particulate.

14. The method of claim 13, wherein the additive includes a flavorant, a pH modifier, an antioxidant, or any combination thereof.

15. The method of any one of the preceding claims, wherein the first solid particulate is a first portion of a solid particulate and the second solid particulate is a second portion of the solid particulate.
